(19) Europäisches Patentamt
European Patent Office
Office européen des brevets

(11) **EP 4 378 319 A1**

(12) **EUROPEAN PATENT APPLICATION**

(43) Date of publication:
**05.06.2024 Bulletin 2024/23**

(21) Application number: **22383164.5**

(22) Date of filing: **01.12.2022**

(51) International Patent Classification (IPC):
*A23J 3/34* (2006.01)   *A23K 20/147* (2016.01)
*A23L 33/18* (2016.01)   *A61K 38/01* (2006.01)
*A61K 38/10* (2006.01)

(52) Cooperative Patent Classification (CPC):
**A23K 20/147; A23C 9/1322; A23C 9/1526;
A23J 3/341; A23L 33/18; A61K 38/012;
A61K 38/10**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM**

(71) Applicant: **Bioiberica, S.A.U.
08389 Palafolls Barcelona (ES)**

(72) Inventors:
• **Segarra, Sergi
08389 Barcelona (ES)**
• **Martínez, Daniel
08389 Barcelona (ES)**

• **De La Torre Gómez, Gisela Carolina
08916 Barcelona (ES)**
• **Bech Serra, Joan Josep
08916 Barcelona (ES)**

(74) Representative: **Pons
Glorieta Rubén Darío 4
28010 Madrid (ES)**

Remarks:
The complete document including Reference
Table(s) and the Sequence Listing(s) can be
downloaded from the EPO website

(54) **COMPOSITION COMPRISING BIOACTIVE PEPTIDES**

(57)     The present invention refers to a composition comprising bioactive peptides, wherein the bioactive peptides are antimicrobial or anti-inflammatory peptides. The invention also relates to the uses of the mentioned composition as well as nutritional compositions and pharmaceutical compositions comprising the bioactive peptides of the invention.

EP 4 378 319 A1

**Description**

The invention relates to a composition that comprises bioactive peptides and its uses, thus the present invention is within the fields of biotechnology and biomedicine.

**BACKGROUND ART**

Animal producers desire to provide a diet for their animals with benefits on health and wellness. Therefore, in order to fulfil this purpose, animal diet must meet the basic nutritional needs, wherein proteins play a key role as essential macronutrients for the proper development and welfare of animals.

Different protein sources, either animal and plant protein sources, have been explored to provide cost-effective and safe feed proteins. There are examples in the prior art that make use of plant-based proteins to produce animal feed compositions (US20080233244A1 and EP2334196A2). There are different sources of plant-based proteins, but the most used source of plant-based protein is soybean due to its high quality of proteins. However, plant-based proteins are characterized by the presence of high levels of anti-nutritional factors such as phytic acid, tannins and alkaloids (Thakur, A., Sharma, V., & Thakur, A. 2019. Int. J. Chem. Stud, 7(1), 2472-2479). These anti-nutritional factors reduce utilization of nutrients especially proteins, vitamins, and minerals, thus preventing optimal exploitation of the nutrients present in a food and decreasing the nutritive value. So, it is necessary to apply strategies to deactivate these compounds, for example using enzymes such as phytases (EP0380343A2). However, the addition of steps for the deactivation of anti-nutritional factor would increase the production cost of plant-based proteins.

In the case of animal protein sources, the protein from milk origins is one of the main options, but the increasing price of protein from milk origin leads the industry to investigate new ingredients for milk replacers (MR). In this regard, the consumption of animal by-products has experienced an increase over the last decade and the utilization of animal by-products as protein source in livestock feed has been successfully carried out without any adverse effect on the animals (Alao, B. O. et al., (2017). Sustainability, 9(7), 1089). Moreover, different enzymatic hydrolysis strategies have been used to improve the digestibility and availability of proteins or peptides from animal by-products. Interestingly, the enzymatic hydrolysis of animal by-products generates peptides that do not only have nutritional value, but also have biological functions like antimicrobial, antioxidant, antihypertensive or immunomodulatory activities (Hou, Y., et al., (2017). Journal of Animal Science and Biotechnology, 8(1), 1-13).

Thus, there is a need to provide new bioactive peptides or combinations of bioactive peptides that can be used in the pharma and alimentary industry.

**DESCRIPTION OF THE INVENTION**

The present invention discloses a composition comprising bioactive peptides having anti-inflammatory and antimicrobial activities. The bioactive peptides generated have great potential as functional foods and nutraceuticals.

The expression "bioactive peptides" refers to polypeptides of a sequence length between 2 and 20 amino acids having biological functions beyond their nutritional value.

In one aspect, the invention refers to a composition comprising the peptides consisting of the amino acid sequences SEQ ID NO: 1, SEQ ID NO: 2, SEQ ID NO: 3, SEQ ID NO: 4, SEQ ID NO: 5 and SEQ ID NO: 6, hereinafter "the composition of the invention", having antimicrobial and anti-inflammatory activities.

SEQ ID NO: 1: TNVPRASVPDGFLS

SEQ ID NO: 2: TNVPRASVPDGFLSEL

SEQ ID NO: 3: DAVEDLESVGK

SEQ ID NO: 4: EGIPPDQQRLIFAGK

SEQ ID NO: 5: TITLEVEPSDTIENVK

SEQ ID NO: 6: VHVVPDQLMAF

In a preferred embodiment, the composition of the invention comprises the following amounts of peptides:

- SEQ ID NO: 1 from 0.01 to 7 pg/$\mu$g of the total amount of peptides in the composition;
- SEQ ID NO: 2 from 0.5 to 25 pg/$\mu$g of the total amount of peptides in the composition;

- SEQ ID NO: 3 from 0.01 to 0.5 pg/μg of the total amount of peptides in the composition:
- SEQ ID NO: 4 from 0.01 to 2 pg/μg of the total amount of peptides in the composition;
- SEQ ID NO: 5 from 0.2 to 0.6 pg/μg of the total amount of peptides in the composition.
- SEQ ID NO: 6 from 0.001 to 5 pg/μg of the total amount of peptides in the composition.

The peptides consisting of the amino acid sequences SEQ ID NO: 1 and SEQ ID NO: 2 and SEQ ID NO: 6 are peptides with anti-inflammatory properties. The term "anti-inflammatory", as used throughout the present description, refers to the capacity of a substance to reduce inflammation.

The capacity of a substance to reduce inflammation can be related to its capacity to regulate the expression or secretion of molecules that control the immune response. In this regard, "cytokines" are small proteins (5-25 kDa) that exert a control on the growth and activity of immune system cells and blood cells. Cytokines are produced by immune cells like macrophages, B lymphocytes, T lymphocytes and mast cells, as well as endothelial cells, fibroblasts, and various stromal cells. Cytokines can be classified into two main groups, proinflammatory and anti-inflammatory cytokines. Proinflammatory cytokines are produced predominantly by activated macrophages and are involved in the up regulation of inflammatory reactions. Some examples of proinflammatory cytokines are interlaukine-2 (IL- 2), interleukine-6 (IL-6), tumor necrosis factor (TNF-α) among others. In contrast, anti-inflammatory cytokines reduce play a role in counterbalancing the pro-inflammatory response. Some example of anti-inflammatory cytokines are interleukin-4 (IL-4), interleukin-10 (IL-10) or interleukin-13 (IL-13). Therefore, an anti-inflammatory substance is able to trigger an up-regulation of anti-inflammatory cytokines or a down regulation of pro-inflammatory cytokines.

The peptides consisting of the amino acid sequences SEQ ID NO: 3, SEQ ID NO: 4 and SEQ ID NO: 5 are antimicrobial peptides. The term "antimicrobial", as used throughout the present description, refers to the capacity of a substance to kill and/or inhibit the growth of microorganisms and especially pathogenic microorganisms.

In a preferred embodiment, the composition of the invention further comprises at least a peptide consisting of the amino acid sequence selected from SEQ ID NO: 7, which is an antimicrobial peptide.

SEQ ID NO: 7: DKEGIPPDQQRLIF

In a preferred embodiment, the composition of the invention comprises the peptide consisting of the amino acid sequence SEQ ID NO: 7 in an amount from 1 to 7 pg/μg total amount of peptides in the composition.

The peptides consisting of the amino acid sequences SEQ ID NO: 1, SEQ ID NO: 2, SEQ ID NO: 3, SEQ ID NO: 4, SEQ ID NO: 5, SEQ ID NO: 6 and SEQ ID NO: 7, are referred as "the peptides of the invention".

In a more preferred embodiment, the composition of the invention comprises the following amounts of peptides:

- SEQ ID NO: 1 from 0.01 to 7 pg/μg of the total amount of peptides in the composition;
- SEQ ID NO: 2 from 0.5 to 25 pg/μg of the total amount of peptides in the composition;
- SEQ ID NO: 3 from 0.01 to 0.5 pg/μg of the total amount of peptides in the composition:
- SEQ ID NO: 4 from 0.01 to 2 pg/μg of the total amount of peptides in the composition;
- SEQ ID NO: 5 from 0.2 to 0.6 pg/μg of the total amount of peptides in the composition;
- SEQ ID NO: 6 from 0.001 to 5 pg/μg of the total amount of peptides in the composition.
- SEQ ID NO: 7 from 1 to 7 pg/μg of the total amount of peptides in the composition;

In some embodiments, the composition of the invention is a nutritional composition, wherein said nutritional composition may be a food or be incorporated into a food or food product intended for human and/or animal consumption.

In the present invention, the term "nutritional composition" refers to any food which, regardless of providing nutrients to the subject who consumes it, beneficially affects one or more functions of the body, so as to leverage and/or provide better health and wellness.

Within the context of the present invention, the term "subject", as used throughout the description, refers to a human being or an animal individual of any sex or age. The term animal refers to any animal except humans. Examples of animals are non-ruminants and ruminants. Ruminant animals include animals such as, but not limited to, sheep, goats, cattle, e.g. beef cattle, cows, and young calves, deer, yank, camel, llama and kangaroo. Non-ruminant animals include monogastric animals, including but not limited to pigs or swine (including, but not limited to, piglets, growing pigs, and sows); poultry such as turkeys, ducks, quail, guinea fowl, geese, pigeons (including squabs) and chicken (including but not limited to broiler chickens), chicks, layer hens (referred to herein as layers)); horses (including but not limited to hotbloods, coldbloods and warm bloods) crustaceans (including but not limited to shrimps and prawns); fish (including but not limited to amberjack, arapaima, barb, bass, bluefish, bocachico, bream, bullhead, cachama, carp, catfish, catla, chanos, char, cichlid, cobia, cod, crappie, sea bream, drum, eel, goby, goldfish, gourami, grouper, guapote, halibut, java) or companion animals or pets (including, but not limited to, dogs, cats or rabbits).

In some embodiments, the composition of the invention is administered to a subject through the diet.

In some embodiments, the nutritional composition of the invention is a nutritional or dietary supplement or an animal feed composition.

In some specific embodiments, the nutritional composition of the invention is nutritional supplement for human beings.

In some specific embodiments, the nutritional composition of the invention is a nutritional supplement for animals as previously defined.

The term "supplement", synonymous with any of the terms "dietary supplement", "nutritional supplement", "food supplement", or "alimentary supplement" or "alimentary complement" refers to products or preparations whose purpose is to supplement the normal diet of a subject consisting of sources of concentrated nutrients or other substances with a nutritional or physiological effect. There is a wide range of nutrients and other elements that may be present in alimentary complements including, among others, vitamins, minerals, amino acids, essential fatty acids, fibre, enzymes, plants and plant extracts. In the present invention, the peptides of the invention are the substances with nutritional and/or physiological effect on the subject.

In some specific embodiments of the invention, the nutritional composition of the invention is a food for specific nutritional and/or functional, biological and/or physiological purposes. In some specific embodiments of the invention, the nutritional composition of the invention is a medicinal food. Examples of food include, but are not limited to, dairy products, vegetable products, meat products, snacks, beverages, cereals, industrial bakery products and biscuits. Examples of dairy products include, but are not limited to, products derived from fermented milk (for example, but not limited to, yogurt or cheese) or non-fermented milk (for example, but not limited to, ice cream, butter, margarine or whey). The vegetable product is, for example, but not limited to, a cereal (such as soy) in any form of presentation, fermented (for example, soy yogurt, oat yogurt, etc.) or unfermented, and a snack. The beverages include, but are not limited to, non-fermented milk. powdered milk, cereals, baked goods, milk-based products (such as milkshakes or smoothies), meat products and beverages.

The term "animal feed composition", as used throughout the description, refers to a composition that is intended for ingestion by an animal as defined above. In the present invention, the animal feed composition comprises the peptides of the invention and feed materials. The term "feed material" refers to a product of vegetable or animal origin, the main purpose of which is to meet the nutritional needs of animals, and which is used for oral feeding. The feed materials include, for example, brans, such as wheat bran, rice bran, barley bran, and millet bran; food processing by products, such as soybean-curd residue, starch pulp, copra meal, sake cake, soy sauce cake, brewer's grains, sweet potato distiller's residue, and juice pulp of fruits and vegetables; cereals, such as corn, rice, wheat, barley, and oat; oil seed meals, such as soybean meal, rapeseed meal, cotton seed meal, linseed meal, sesame meal, and sunflower meal; animal origin feeds, such as fish meal, casein, dried skim milk, dried whey, meat and bone meal, meat meal, feather meal, and blood meal; leaf meals, such as alfalfa meal; and the like , so long as it does not have influences upon the effect of the invention.

In a preferred embodiment, the composition of the invention is a pharmaceutical or veterinary pharmaceutical composition wherein said pharmaceutical composition further comprises at least a pharmaceutically acceptable excipient and/or a carrier.

The expression "veterinary pharmaceutical composition" refers to a pharmaceutical composition for veterinary use.

The term "excipient" refers to a substance that aids in the absorption of any of the components of the composition of the present invention, stabilizes said components, or aids in the preparation of the composition. Thus, the excipients could have the function of keeping the components together, such as starches, sugars, or cellulose, sweetening function, colorant function, drug protection function such as to isolate it from air and/or humidity, function filling of a pill, capsule or any other form of presentation such as dibasic calcium phosphate, disintegrating function to facilitate the dissolution of the components and their absorption in the intestine, without eliminating other types of excipients not mentioned in this paragraph. Therefore, the term "excipient" is defined as that material that, included in the "galenic forms", is added to the active ingredients or their associations to enable their preparation and stability, modify their organoleptic properties or determine the physical-chemical properties of the pharmaceutical or veterinary composition and its bioavailability. Furthermore, the excipient is pharmacologically or veterinary acceptable, that is, the excipient is permitted and evaluated such that it does not cause harm to the organisms to which it is administered.

The "vehicle" or "carrier" is preferably an inert substance. Carrier functions are to facilitate the incorporation of other components or compounds, allow better dosage and administration and/or give consistency and form to the pharmaceutical composition. Therefore, the carrier is a substance used in the drug to dilute any of the components or compounds of the pharmaceutical composition of the present invention to a given volume or weight; or that even without diluting these components or compounds, it is able to allow better dosage and administration and/or give consistency and form to the drug. When the presentation is liquid, the pharmaceutically acceptable carrier is the diluent. The carrier can be natural or unnatural. Examples of pharmaceutically acceptable carriers include, without being limited thereto, water, salt solutions, alcohol, vegetable oils, polyethylene glycols, gelatine, lactose, starch, amylose, magnesium stearate, talc, surfactants, silicic acid, viscous paraffin, perfume oil, monoglycerides and diglycerides of fatty acids, fatty acid esters petroetrals, hydroxymethylcellulose, polyvinylpyrrolidone and the like.

Furthermore, the excipient and the carrier must be pharmacologically acceptable, i.e., the excipient and the carrier are permitted and evaluated so as not to cause damage to the subject to whom it is administered.

In each case the presentation of the composition will be adapted to the type of administration used. The composition of the invention can be formulated into solid, semisolid, or liquid preparations, such as tablets, capsules, powders, granules, solutions, suppositories, gels or microspheres. In a particular embodiment, the composition of the invention is formulated in liquid form or in solid form.

In another particular embodiment, the solid formulation is selected from the group consisting of tablets, lozenges, sweets, chewable tablets, chewing gums, capsules, sachets, powders, gels, pellets, granules, coated particles or coated tablets, tablet, pills, troches, gastro-resistant tablets and capsules and dispersible strips and films.

In another particular embodiment, the liquid formulation is selected from the group consisting of oral solutions, suspensions, droplets, emulsions and syrups.

In some embodiments of the invention, the administration regime of the composition of the invention is at least once daily; twice daily; or three times a day, one with each main food intake (breakfast and/or lunch and/or dinner).

Since the composition of the invention has beneficial biological activities, another aspect, refers to the composition of the invention as a medicament.

In another aspect, the invention refers to the composition of the invention for use in the treatment and/or prevention of a disease or condition caused by a microbial pathogen, hereinafter "the antimicrobial use of the invention".

The terms "treat," "treating" or "treatment," and other grammatical equivalents as used throughout the present description, include alleviating, inhibiting or reducing symptoms, reducing or inhibiting severity of, reducing incidence of, prophylactic treatment of, reducing or inhibiting recurrence of, preventing, delaying onset of, delaying recurrence of, abating or ameliorating a disease or condition symptoms, ameliorating the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition.

The terms "prevent," "preventing" or "prevention," and other grammatical equivalents as used throughout the present description, include preventing additional symptoms, preventing the underlying metabolic causes of symptoms, inhibiting the disease or condition, e.g., arresting the development of the disease or condition and are intended to include prophylaxis. The terms further include achieving a prophylactic benefit. For prophylactic benefit, the compositions are optionally administered to an individual at risk of developing a particular disease, to an individual reporting one or more of the physiological symptoms of a disease, or to an individual at risk of reoccurrence of the disease.

The term "microbial pathogen", as used in the present invention, refers to any microorganism which may colonize and cause a disease in a subject as defined before. In a preferred embodiment the microbial pathogen is a Gram-positive or Gram-negative bacterium.

The term "Gram-positive bacteria", as used throughout the present description, refers to Gram-positive bacteria which are known and/or can be identified by the presence of certain cell wall and/or cell membrane characteristics and/or by staining with Gram staining. Gram positive bacteria are known and can readily be identified and may be selected from, but are not limited to, the genera *Listeria, Staphylococcus, Streptococcus, Enterococcus, Mycobacterium, Corynebacterium,* and *Clostridium,* and include all recognized or unrecognized species or strains thereof. In a preferred embodiment the microbial pathogen is *Staphylococcus aureus.*

The term "Gram-negative bacteria" generally refers to bacteria which produce a crystal violet stain that is decolorized in Gram staining, i. e. they do not retain crystal violet dye in the Gram staining protocol. Gram negative bacteria are known and can readily be identified and may be selected from, but are not limited to, the genera *Pseudomonas, Klebsiella, Enterobacter, Escherichia coli, Citrobacter, Salmonella, Yersinia, Shigella, Proteus, Pseudomonas, Vibrio, Legionella, Haemophilus, Bordetella, Brucella, Campylobacter, Neisseria and Branhamella,* including all recognized or unrecognized species or strains thereof. In a preferred embodiment the Gram-negative bacterium is *Escherichia coli.*

In another aspect, the invention refers to the composition of the invention for use in the treatment and/or prevention of an inflammatory condition, hereinafter the anti-inflammatory use of the invention.

The expression "inflammatory diseases or condition", as used in the present description, refers to disease or condition associated with a disbalance between the pro-inflammatory and anti-inflammatory activity of the immune system, wherein such pro-inflammatory activity can be enhanced (i.e. up regulation of proinflammatory cytokines) or the anti-inflammatory activity can be inhibited (i.e. down regulation of anti-inflammatory cytokines).

In a preferred embodiment, the inflammatory disease or condition can be selected from, without limitation, cardiovascular disease, cancer, allergy, diabetes, digestive disease, neurological disease, or autoimmune disease.

In preferred embodiments, the cardiovascular disease is selected from, but not limited to, atherosclerosis, peripheral vascular diseases, cerebrovascular disease (i.e., stroke), hypertension, heart failure, rheumatic heart disease, bacterial endocarditis, cardiomyopathy, pulmonary circulation diseases, vein and lymphatic diseases, or other diseases significantly affecting cardiovascular function.

In preferred embodiment, cancer is selected from, but not limited to, prostate cancer, non-small cell lung carcinoma; ovarian cancer, breast cancer, melanoma, gastric cancer, colorectal cancer, brain cancer, metastatic bone disorder, pancreatic cancer, bladder cancer, hepatocellular cancer, liver cancer, adenocarcinoma of the lung, oesophageal squamous cell carcinoma, CNS tumors (e.g., Glioblastoma and neuroblastoma), haematological tumors or a lymphoma.

The term "allergy", as used throughout the description, means a disorder characterized by excessive activation of mast cells and basophils by IgE. In certain instances, the excessive activation of mast cells and basophils by IgE results (either partially or fully) in an inflammatory response. In certain instances, the inflammatory response is local. In certain embodiments, the inflammatory response results in the narrowing of airways (i.e., bronchoconstriction). In certain embodiments, the inflammatory response results in inflammation of the nose (i.e., rhinitis). In certain instances, the inflammatory response is systemic (i.e., anaphylaxis).

The term "diabetes", as used throughout the description, refers to a chronic, metabolic disease characterized by elevated levels of blood glucose (or blood sugar), which leads over time to serious damage to the heart, blood vessels, eyes, kidneys and nerves, wherein diabetes can be such as diabetes mellitus type 1 or type 2.

In a preferred embodiment the digestive disease is selected from, but not limited to ulcerative colitis, hepatitis, pancreatitis, inflammatory bowel disease or other diseases significantly affecting gastrointestinal (GI) function.

In a preferred embodiment neurological disease is selected from, but not limited to Alzheimer's disease, Parkinson's disease, epilepsy, ataxia, motor neuron disease, dementia or other diseases significantly affecting neurological function.

In a preferred embodiment autoimmune diseases is selected from, but not limited to, autoimmune hemolytic syndromes, autoimmune hepatitis, autoimmune neuropathy, autoimmune ovarian failure, autoimmune orchitis, autoimmune thrombocytopenia, reactive arthritis, ankylosing spondylitis, silicone implant associated autoimmune disease, Sjogren's syndrome, systemic lupus erythematosus (SLE), vasculitis syndromes (e.g., giant cell arteritis, Behcet's disease & Wegener's granulomatosis), vitiligo, secondary hematologic manifestation of autoimmune diseases (e.g., anemias), drug-induced autoimmunity, Hashimoto's thyroiditis, hypophysitis, idiopathic thrombocytic purpura, metal-induced autoimmunity, myasthenia gravis, pemphigus, autoimmune deafness (e.g., Meniere's disease), Goodpasture's syndrome, Graves' disease, HIV-related autoimmune syndromes or Guillain-Barre disease.

The antimicrobial use of the invention and the anti-inflammatory use of the invention are directed to a subject, wherein the subject is a human, or an animal as defined before.

**Examples**

**Example 1. Obtention and identification of bioactive peptides**

The peptides of the invention were obtained by digestion with alcalase in a process that uses as starting material a porcine mucosa sample. Two protein hydrolysates were obtained: the product 1, in the form of liquid, was the resulting product of enzymatic hydrolysis of porcine mucosa sample. Next, the product 1 was dried by spray drying to obtain the product 2 in form of a powder.

Mass spectrometry-based proteomic and peptidomics approach was used in order to identify, and monitoring the peptide of interest present in product 1 and 2.

First, the peptide fraction of products 1 and 2 were obtained as follows:

Extraction. The peptide fraction was extracted with 10% formic acid and hard vortex. Sample was sonicated with an ultra-tub sonicator. The sonication was done with cold to avoid the sample to heat. Centrifuge at max speed 10 minutes and we keep the supernatant.

Peptides purification and desalting. Samples were desalted using Sep-pack 200mg from waters. Desalted peptides were dried in the speedvac. Peptide resuspended in 0.1%TFA (trifluoroacetic solution).

The peptide fractions of products 1 and 2 were analysed by mass spectroscopy using the following conditions (LC-MS):

LC conditions

injected volume 2.4 μL (1.8 μg) + 1.7 μL stock AQUA Heavy peptide
1.37 μL (1.04 μg) + 1.7 μL stock AQUA Heavy peptide
Chromatograph Thermo Scientific Dionex Ultimate 3000
Trap column μ-precolumn 300 μm i.d. × 5 mm PepMap100, 5μm, 100 Å, C18 (Thermo Scientific)
Analytical column NanoEase MZ HSS T3 column (75 μm × 250 mm, 1.8 μm, 100 Å) (Waters)

Eluents :

A. H2O 0.1% formic acid
B. CH3CN 0.1% formic acid

The gradient was used to run all samples were:
3% to 35% of B in 60 min + 35% to 50% in 5 min + 50% to 85% in 2 min at 250 nL/min flow rate.

LC-MS coupling:

Source Advion Triversa Nanomate (Advion BioSciences, Ithaca, NY, USA)
nESI through chip technology
Spray voltage 1.7 kV
Delivery pressure 0.5 psi
Mode Positive

MS conditions

Mass spectrometer Orbitrap Fusion Lumos™ Tribrid (Thermo Scientific)
Ion transfer tube temp.: 275oC
RF Lens 30%
Acquisition mode MS1 > tMS
m/z range (MS1) 300-1000 a.m.u
AGC (MS1) 4x105
Detector type OT (120 k)
Scan 2 tMS
Isolation window 1.6
Fragmentation method HCD
Collision energy 28%
AGC 1.25x105
Targeted mode Mass list table (attached excel)
Detection Orbitrap (30 k)
Instrument acquisition software Xcalibur software vs 4.2.28.14 (Thermo Scientific)

Table 1. Peptides identified by peptidomes and proteomics in each product.

| Product | Peptides |
|---------|----------|
| 1 | 961 |
| 2 | 1134 |

All the data were processed by using MaxQuant and PEAKS softwares.
To determine the biological activity of all the identified peptides, the inventors first explored the main databases with bioactive peptides in order to create a list of peptides and their reported biological activities. The names of the databases used to create the final database are shown in Table 2.

Table 2. List of public databases used to create a bioactive peptides database.

| Name | URL | Biological activity |
|---|---|---|
| Antimicrobial peptide database APD2 | http://aps.unmc.edu/AP/main.php | Antimicrobial |
| Neuropedia | http://proteomics.ucsd.edu/Softwar e/ NeuroPedia/ | Neuropeptides |
| StraPep | http://isyslab.info/StraPep/ | Antimicrobial Toxic venom peptide Cytokine/growth factor (immunomodulation) Hormone Neuropeptide Other |
| AHTPDB | https://webs.iiitd.edu.in/raghava/aht pdb/ | Antihypertensive |
| BIOPEP-UWM | http://www.uwm.edu.pl/biochemia/i ndex.php/pl/biopep | Antioxidant |

This homemade database was interrogated with all the peptides identified in Table 1 to classified their biological fuctions. To promote the bioactive peptide prediction and discovery from all peptides identified (Table 1) a machine-learning-based algorithms was designed and developed to increase the prediction accuracy. In this regard, three different classifiers were implemented using the Random Forests algorithm to predict peptides with antimicrobial and anti-inflammatory activity. The algorithms were trained using the antimicrobial and anti-inflammatory peptides present in the home-made database (see Table 2 above).

The classifiers were trained to classify peptides from 7-25 amino acids length, therefore, the peptides out of this range were discarded. A summary of all the peptides predicted and their biological activities is shown in table 3 .

Table 3. Peptides and biological activities.

| Peptides | Biological activity |
|---|---|
| SEQ ID NO: 1: TNVPRASVPDGFLS | Cytokine/growth (immunomodulation) factor |
| SEQ ID NO: 2: TNVPRASVPDGFLSEL | Cytokine/growth (immunomodulation) factor |
| SEQ ID NO: 3: DAVEDLESVGK | Antimicrobial |
| SEQ ID NO: 4: EGIPPDQQRLIFAGK | Antimicrobial |
| SEQ ID NO: 5: TITLEVEPSDTIENVK | Antimicrobial |
| SEQ ID NO: 6: VHVVPDQLMAF | Cytokine/growth (immunomodulation) factor |
| SEQ ID NO: 7: DKEGIPPDQQRLIF | Antimicrobial |
| *Cytokine/growth factor peptides were predicted as anti-inflammatory peptides | |

Peptides of SEQ ID NO: 1, SEQ ID NO: 2, SEQ ID NO: 3, SEQ ID NO:4 , SEQ ID NO: 5 and SEQ ID NO: 6 were present in product 1 and 2. Peptide of SEQ ID NO: 7 was only present in product 1.

**Example 2. Quantification of peptides**

The absolute amount of peptides of table 3 was determined in products 1 and 2 (Table 4). Heavy labeled synthetic peptides (AQUA peptides) corresponding to the peptides of table 3 were spiked in the peptide fractions of products 1 and 2 and mass spectrometry analisis was performed as in example 1. The analysis of the targeted Mass spectrometry data was performed using Skyline v.19.1.0.193, an open source software (Sherrod SD et al. J Proteome Res. 2012 Jun 1; 11(6):3467-79). The pg of peptides of table 3 by $\mu$g of the total amount of peptides in the composition was caluclated according to following formula:

$$\text{Amount of peptide} = (L / H) \times H$$

Where L/H is the ratio Light/Heavy peak areas obtained in Skyline; H is the amount of heavy peptide in pg injected on column.

Table 4. Targeted peptides amount expressed in average pg Peptide / $\mu$g of total amount of peptides.

| Peptides | Product 1 | Product 2 |
|---|---|---|
| SEQ ID NO: 1 | 1.4596 | 0.1898 |
| SEQ ID NO: 2 | 8.0500 | 0.9224 |
| SEQ ID NO: 3 | 0.1626 | 0.1939 |
| SEQ ID NO: 4 | 0.2637 | 0.1852 |
| SEQ ID NO: 5 | 0.3594 | 0.4327 |
| SEQ ID NO: 6 | 0.0310 | 0.0054 |
| SEQ ID NO: 7 | 2.3513 | Not detected |

**Claims**

1. A composition the peptides consisting of the amino acid sequences SEQ ID NO: 1, SEQ ID NO: 2, SEQ ID NO: 3, SEQ ID NO: 4 , SEQ ID NO: 5 and SEQ ID NO: 6

2. The composition according to claim 1, wherein the amounts of the peptides are:

    - SEQ ID NO: 1 from 0.01 to 7 pg/$\mu$g of the total amount of peptides in the composition;
    - SEQ ID NO: 2 from 0.5 to 25 pg/$\mu$g of the total amount of peptides in the composition;
    - SEQ ID NO: 3 from 0.01 to 0.5 pg/$\mu$g of the total amount of peptides in the composition:
    - SEQ ID NO: 4 from 0.01 to 2 pg/$\mu$g of the total amount of peptides in the composition; and
    - SEQ ID NO: 5 from 0.2 to 0.6 pg/$\mu$g of the total amount of peptides in the composition.
    - SEQ ID NO: 6 from 0.001 to 5 pg/$\mu$g of the total amount of peptides in the composition.

3. The composition according to claim 1 or 2, further comprising the peptide consisting of the amino acid sequence SEQ ID NO: 7.

4. The composition according to any one of claims 1 to 3, wherein composition of the invention comprises the peptide consisting of the amino acid sequence SEQ ID NO: 7 in an amount from 1 to 7 pg/$\mu$g total amount of peptides in the composition.

5. The composition according to any one of claims 1 to 4, wherein the composition is a nutritional composition.

6. The composition according to claim 5, wherein the nutritional composition is a nutritional supplement or an animal feed composition.

7. The composition according to any one of claims 1 to 4 that is a pharmaceutical or veterinary pharmaceutical composition wherein said pharmaceutical composition further comprises at least a pharmaceutically acceptable excipient and/or a carrier.

8. The composition according to any one of claims 1 to 4, or 7, for use as a medicament.

9. The composition according to any one of claims 1 to 4, or 7, for use in the treatment and/or prevention of a disease or condition caused by a microbial pathogen.

10. The composition according to any one of claims 1 to 4, or 7, for use in the treatment and/or prevention of an inflammatory disease or condition.

Europäisches
Patentamt

European
Patent Office

Office européen
des brevets

# EUROPEAN SEARCH REPORT

Application Number

EP 22 38 3164

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 607 840 A (VAN GORP CORNELIUS L [US] ET AL) 4 March 1997 (1997-03-04) * column 4, line 35 - line 36; claim 1; example 4 * * column 6, line 2 - line 6 * ----- | 1-10 | INV. A23J3/34 A23K20/147 A23L33/18 A61K38/01 A61K38/10 |
| X | GONZÁLEZ-SOLÉ FRANCESC ET AL: "Porcine Digestible Peptides (PDP) in Weanling Diets Regulates the Expression of Genes Involved in Gut Barrier Function, Immune Response and Nutrient Transport in Nursery Pigs", ANIMALS, vol. 10, no. 12, 10 December 2020 (2020-12-10), page 2368, XP093039643, DOI: 10.3390/ani10122368 * Simple Summary * ----- | 1-10 | |
| X | FRIKHA M. ET AL: "Hydrolyzed porcine mucosa in broiler diets: Effects on growth performance, nutrient retention, and histomorphology of the small intestine", POULTRY SCIENCE, vol. 93, no. 2, 1 February 2014 (2014-02-01), pages 400-411, XP093039646, Oxford ISSN: 0032-5791, DOI: 10.3382/ps.2013-03376 * abstract * ----- | 1-10 | TECHNICAL FIELDS SEARCHED (IPC) A23J A23L A61K C11C A23K |
| X | WO 2011/038149 A2 (CAROLUS THERAPEUTICS INC [US]; BERNHAGEN JURGEN [DE] ET AL.) 31 March 2011 (2011-03-31) | 8-10 | |
| A | * claim 30; sequence 186 * ----- | 1-7 | |

The present search report has been drawn up for all claims

| Place of search | Date of completion of the search | Examiner |
|---|---|---|
| The Hague | 18 April 2023 | Du, Mingliu |

EPO FORM 1503 03.82 (P04C01)

## ANNEX TO THE EUROPEAN SEARCH REPORT
## ON EUROPEAN PATENT APPLICATION NO.

EP 22 38 3164

This annex lists the patent family members relating to the patent documents cited in the above-mentioned European search report.
The members are as contained in the European Patent Office EDP file on
The European Patent Office is in no way liable for these particulars which are merely given for the purpose of information.

18-04-2023

| Patent document cited in search report | | Publication date | Patent family member(s) | | Publication date |
|---|---|---|---|---|---|
| US 5607840 | A | 04-03-1997 | AU | 5681094 A | 22-06-1994 |
| | | | US | 5607840 A | 04-03-1997 |
| | | | WO | 9412524 A1 | 09-06-1994 |
| WO 2011038149 | A2 | 31-03-2011 | AU | 2010298249 A1 | 19-04-2012 |
| | | | BR | 112012006468 A2 | 09-08-2016 |
| | | | CA | 2773978 A1 | 31-03-2011 |
| | | | CN | 102725311 A | 10-10-2012 |
| | | | EP | 2480579 A2 | 01-08-2012 |
| | | | KR | 20120105429 A | 25-09-2012 |
| | | | WO | 2011038149 A2 | 31-03-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20080233244 A1
- EP 2334196 A2
- EP 0380343 A2

### Non-patent literature cited in the description

- **THAKUR, A. ; SHARMA, V. ; THAKUR, A.** *Int. J. Chem. Stud,* 2019, vol. 7 (1), 2472-2479
- **ALAO, B. O. et al.** *Sustainability,* 2017, vol. 9 (7), 1089
- **HOU, Y. et al.** *Journal of Animal Science and Bio-technology,* 2017, vol. 8 (1), 1-13
- **SHERROD SD et al.** *J Proteome Res.,* 01 June 2012, vol. 11 (6), 3467-79